# EUROPEAN PATENT APPLICATION

(11) **EP 1 712 562 A1**
(43) Date of publication of application: **18.10.2006**
(21) Application number: 04808172.3
(22) Date of filing: 27.12.2004
(51) Int. Cl.: C07K 14/805

(54) **PROCESS FOR PURIFICATION OF HEMOGLOBIN**

(30) Priority: 26.12.2003 JP 2003433548
(71) Applicant: Oxygenix Co., Ltd., Tokyo 105-0001 (JP); WASEDA UNIVERSITY, Shinjuku-ku, Tokyo 169-8050 (JP)
(72) Inventor: TAKEOKA, Shinji, Tokyo 1580094 (JP); TSUCHIDA, Eishun, Tokyo 1770053 (JP); SOU, Keitaro, Tokyo 1710031 (JP)
(74) Representative: Woods, Geoffrey Corlett
(86) International application number: PCT/JP2004/019822
(87) International publication number: WO 2005/063814

(57) **Abstract**

The present invention provides a method for purifying hemoglobin from red blood cells, wherein the red blood cells are deep-frozen and preserved, and then purified.

## Description

### TECHNICAL FIELD

The present invention relates to a method for purifying hermoglobin (Hb) from red blood cells, and in particular to a method including a step of deep-freezing and preserving, in a frozen state, red blood cells in order to obtain Hb stably at a high yield without causing any specific denaturation or deterioration of Hb.

### BACKGROUND ART

Hemoglobin (Hb) isolated and purified from red blood cells is usable as a material of a red blood cell substitute. Therefore, a method for stably obtaining a hemoglobin material fulfilling certain standards is desired. Usually, red blood cells are refrigerated and preserved (4°C). However, red blood cells extracted from a living body by blood extraction are deteriorated as time passes due to various chemical changes (hemolysis, membrane component denaturation, metabolite production, Hb oxidation, germ proliferation, etc.). Therefore, it is difficult to obtain highly pure Hb.

In particular, Hb responsible for oxygen transport can dissociate oxygen from the bond only when the heme iron center is divalent (Fe²⁺). Once the heme iron center is oxidized to be trivalent (Fe³⁺, metHb) by some external factor such as, for example, electron migration (automatic oxidation) accompanying oxygen dissociation or active oxygen, the Hb loses the oxygen-binding capability thereof and cannot be used as an Hb material (referred to as "change into methemoglobin"). In red blood cells, methemoglobin (metHb) is reduced rapidly by reducing enzymes, and active oxygen which changes hemoglobin into methemoglobin is erased by enzymes such as, for example, superoxide dismutase and catalase. As a result, the metHb concentration in red blood cells is kept low. However, this function is also deteriorated while the red blood cells are preserved in a refrigerated state (4°C), and metHb is progressively accumulated. For these reasons, a method for purifying Hb from red blood cells needs to include a step of transferring Hb into an environment, in which Hb is unlikely to be oxidized, by a simple and low-cost technique within a certain time period after the blood extraction. Before Hb is transferred into such an environment, it is indispensable to set conditions for such an environment. Some methods for purifying Hb from red blood cells are known (Japanese Laid-Open Patent Publication No. 9-12598, Japanese National-Phase PCT Laid-Open Publication No. 2002-520338). These known methods do not include any step regarding the handling of red blood cells, which is a precondition for transferring Hb into the environment, and do not set any conditions, needless to say. In addition, in the case where blood extracted from a human body is used after the preservation time limit therefor (three weeks in Japan) expires, quality control is more difficult after the expiration. Deep-freezing of a substance having a cell structure like red blood cells has been considered to be difficult without adding a protection agent (Japanese National-Phase PCT Laid-Open Publication No. 2002-516254). Moreover, a protection agent requires a complicated procedure to be added or removed and thus is not suitable for purification of a large volume of Hb.

### DISCLOSURE OF THE INVENTION

In such a situation, the properties of the obtained purified Hb are significantly influenced by the preservation conditions or preservation term of red blood cells used as a material, which causes the problem that the quality of the purified Hb is not stable. In order to stably obtain purified Hb, it is necessary to establish an Hb purification method including a step of controlling red blood cells without changing the state thereof.

The present inventors, as a result of conducting active studies in light of the above circumstances, found that Hb included in red blood cells is stably preserved by deep-freezing and preserving red blood cells before purification. With the condition that washed red blood cells are deep-frozen in this manner, the reduction in the Hb yield can be prevented. The present inventors found that according to a method for purifying Hb including such a step, the term in which red blood cells are usable is significantly extended and also purified Hb having stable properties are obtained, and thus completed the present invention.

The present invention is directed to a method for purifying hemoglobin from red blood cells, wherein the red blood cells are deep-frozen and preserved, and then purified.

In the method, the step of deep-freezing and preserving the red blood cells may comprise a step of preserving the extracted red blood cells at 4°C through 10°C, a step of washing the red blood cells, a step of deep-freezing the washed red blood cells, and a step of preserving the deep-frozen red blood cells in a frozen state.

The term from the red blood cells are extracted until deep-frozen is, for example, 1 through 60 days.

The step of deep-freezing the red blood cells and the step of preserving the red blood cells in the frozen state are performed at a temperature of, for example, -60°C or lower.

The present invention provides a method for purifying hemoglobin from red blood cells, wherein the red blood cells are deep-frozen and preserved, and then purified. A method for purifying hemoglobin according to the present invention significantly extends the term in which red blood cells are usable, and also provides purified Hb having stable properties.

Hereinafter, the present invention will be described in detail.

In the present invention, red blood cells are obtained from blood derived from various animals (e.g., human, bovine, equine, or porcine bodies, but not limited thereto). Preferably, a red blood cell dispersant, which has most of the white blood cells, platelets and plasma component removed by centrifugation, ultrafiltration, membrane filtration or a combination thereof after blood extraction, is used. Red cell M.A.P. is a red cell concentrate preparation obtained by removing the plasma component from donated blood by centrifugation and adding a MAP solution as a stabilizer to the resultant substance. Red cell M.A.P. for which the preservation time limit has expired (21 days after blood extraction) is used. A "MAP solution" mainly contains mannitol, adenine and crystalline sodium dihydrogen phosphate. Red cell M.A.P. is obtained by replacing CPD (citrate phosphate dextrose), which is an anticoagulant in the red cell concentrate preparation, with a MAP solution in order to improve the survival rate of red blood cells.

A step of refrigerating and preserving the red blood cells is preferably performed at 4 through 10°C, and any of usual techniques including refrigerator, blood cooler and preserver is usable. ' A temperature of lower than 4°C is not preferable because when the red blood cells are deep-frozen after being preserved at such a temperature, there occur problems that, for example, metHb (methemoglobin) is accumulated due to disfunction of the reducing enzyme and that the Hb yield is lowered in the subsequent washing step due to hemolysis of the red blood cells. A temperature of higher than 10°C is not preferable either because the red blood cells are acceleratingly deteriorated due to production of metHb, production of various metabolites, proliferation of germs, or the like.

For a step of washing the red blood cells, any method is usable as long as the red blood cells are separated from remaining white blood cells, platelets and the plasma component, and from water-soluble substances produced during the preservation. For example, centrifugation, ultrafiltration, membrane filtration, or a combination thereof is usable. After being washed, the red blood cells may be dispersed in a red blood cell pellet obtained by centrifugation or in a solution controlled to have a crystalloid osmotic pressure equal to that of the red blood cells. Any additive which cannot be easily removed in the step of purifying hemoglobin should be avoided. In the case of red cell M.A.P., it is preferable that after the preservation time limit expires, the following procedure is repeated twice, three times or four times at a blood center or a hospital: physiological saline is injected into the blood bags and high-rate centrifugation is performed for each bag, thereby removing a supernatant containing buffy coat (a layer of white blood cells and platelets). As a result, a red cell concentrate is obtained.

For a step of deep-freezing the washed red blood cells at -60°C or lower, any of techniques including immersion in an appropriate refrigerant, atomization of a refrigerant, a refrigerator, and a cooler is usable. For rapid deep-freezing, liquid nitrogen, a freezer of -80°C, or deep-freezing by atomization is useful. In the case of red cell M.A.P., each bag may be deep-frozen. In the present invention, the term from the red blood cells are extracted until deep-frozen is, for example, 1 through 60 days.

For a step of preservation in a frozen state, any of techniques including immersion in an appropriate refrigerant, atomization of a refrigerant, and a freezer is usable. In the case where the bond water is not deep-frozen when the high-order structure of Hb is changed by the deep-freezing, production of metHb may be undesirably promoted. Therefore, preservation is preferably performed at -60°C or lower. In the case of red cell M.A.P., the red blood cells in the deep-frozen state may be collected to the site of hemoglobin purification.

For a method for purifying hemoglobin, any available method is usable. For example, a stroma-free hemoglobin solution may be obtained by hemolyizing the blood by a usual technique and removing only the stroma component by centrifugation or ultrafiltration; or a purified hemoglobin solution may be obtained by isolating hemoglobin.

Regarding the above-described method for purification, ultrafiltration may be performed using an ultrafiltration membrane having an ultrafiltering molecular weight of 70 k through 1000 kDa, preferably 500 kDa. It is preferable that the solution deprived of the stroma component is optionally degassed or exposed to carbon monoxide gas to be converted into carbonyl Hb by 98% or more. Next, the Hb solution is stirred in a constant temperature bath and then centrifuged at a predetermined rate, thereby removing precipitated denatured impure protein. By ultrafiltering the resultant solution, a purified hemoglobin solution can be obtained as a filtered solution.

Hereinafter, the present invention will be described in more detail by way of examples, but the present invention is not limited to these examples.

### [Example 1] Comparison of the preservation states of red cell M.A.P.

From a bag (200 ml) of red cell M.A.P. provided by the Japanese Red Cross Blood Center for which the preservation time limit has expired (27 days after blood extraction), about 140 mL was retrieved, put into a centrifugal tube, and subjected to centrifugation at 4°C (2,000 x g, 15 min.), thereby removing a supernatant containing buffy coat. Then, 70 mL of physiological saline was added thereto, stirred, and washed by centrifugation. This procedure was repeated four times to obtain a red cell concentrate. The red cell concentrate was divided into polyethylene containers as samples each of 3 mL, and the samples were respectively preserved in a refrigerator (4°C), a freezer (-20°C) and a deep-freezer (-80°C). A predetermined number of days later, the samples were defrosted in a refrigerator of 4°C, and the methemoglobin content (changing ratio to methemoglobin) of each sample was measured by the cyanomethemoglobin method. Table 1 shows the results. In the case of preservation in the refrigerator (4°C) and the freezer (-20°C), the changing ratio to methemoglobin was increased as time passed. Especially, at -20°C, a conspicuous increase of 37.1% was recognized 14 days after freezing. By contrast, in the case of preservation in the deep-freezer (-80°C), the changing ratio was not increased even 3 months later.

**Table 1: Red blood cell preservation temperature vs. changing ratio into methemoglobin**

| | | | | | | |
|---|---|---|---|---|---|---|
| Term after blood extraction (days) | 27 | 28 | 41 | 58 | 87 | 117 |
| Freezing term (days) | 0 | 1 | 14 | 31 | 60 | 90 |
| Preservation temperature | Changing ratio into methemoglobin (%) | | | | | |
| 4°C | 0 | 0 | 0.7 | 2.8 | 15.9 | 35.5 |
| -20°C | 0 | 5.1 | 37.1 | N.D. | N.D. | N.D. |
| -80°C | 0 | 0.7 | 0.7 | 0.9 | 0.8 | 0.9 |

### [Example 2] Red cell M.A.P. preservation temperature in a refrigerated state

Red cell M.A.P. provided by the Japanese Red Cross Blood Center, for which the preservation time limit has expired, was refrigerated and preserved at 4°C. 27 days after the blood extraction, the red cell M.A.P. was divided into samples and preserved in a cooler at 4°C, 10°C, 15°C and 20°C. 58 days after the blood extraction, the methemoglobin content (changing ratio to methemoglobin) of each sample was measured by the cyanomethemoglobin method. In the case of preservation at 4°C and 10°C, the changing ratios were respectively 2.8% and 6.4%. By contrast, in the case of preservation at 15°C and 20°C, the changing ratios were as high as 13.2% and 18.3%. In the case of preservation at 10°C or lower, the changing ratios were 10% or less until at least 60 days after the blood extraction.

### [Example 3] Preservation term of red cell M.A.P. in a refrigerated state

Red cell M.A.P. provided by the Japanese Red Cross Blood Center, for which the preservation time limit has expired, was refrigerated and preserved in a bag at 4°C. 27 days, 41 days, 58 days and 87 days after the blood extraction, the red cell M.A.P. was retrieved in an amount of about 140 mL, transferred to a centrifugal tube, and subjected to centrifugation (2,000 x g, 15 min.), thereby removing a supernatant containing buffy coat. Then, 70 mL of physiological saline was added thereto, stirred, and then washed by centrifugation. This procedure was repeated three times to obtain a red cell concentrate. The red cell concentrate was put into polyethylene containers as samples each of 3 mL, and preserved in a deep-freezer (-80°C). A predetermined number of days later, the samples were defrosted in a refrigerator of 4°C, and the methemoglobin content (changing ratio to methemoglobin) of each sample was measured by the cyanomethemoglobin method. Table 2 shows the results. In the case of preservation at 4°C, it is clearly shown that the changing ratio after defrosting was increased as the preservation term extended. Based on these results, it was determined that with the upper limit of the changing ratio after defrosting being set at 10%, an appropriate term after the blood extraction until deep-freezing is within 60 days.

**Table 2: Influence of the blood red cell preservation term in a refrigerated state on the post-defrosting changing ratio into methemoglobin**

| | | | | | | |
|---|---|---|---|---|---|---|
| Freezing term (days) | 0 | 1 | 7 | 14 | 31 | 60 |
| Preservation term in a refrigerated state (days) | Changing ratio into methemoglobin (%) | | | | | |
| 27 | 0 | 0.7 | 0.8 | 0.7 | 0.9 | 0.9 |
| 41 | 0.7 | 4.6 | 4.2 | 4.4 | 4.4 | 4.6 |
| 58 | 2.8 | 8.3 | 8.5 | 8.8 | 9.0 | 9.5 |
| 87 | 12.3 | 20.5 | 21.2 | 21.8 | 22.3 | 22.6 |

### [Example 4] Method for purifying Hb from deep-frozen red cell M.A.P.

A bag (400 ml) of red cell M.A.P. provided by the Japanese Red Cross Blood Center (28 days after blood extraction) was immersed in liquid nitrogen for 10 minutes to be deep-frozen, preserved in a freezer (-80°C) for 60 days, and defrosted in a refrigerator (4°C). The defrosted red blood cells were subjected to centrifugation at 4°C (2,000 x g, 15 min.). The supernatant was dark red, which demonstrates that the red blood cells were hemolyzed by the operation of deep-freezing and melting. After the supernatant containing buffy coat was removed, 140 mL of physiological saline was added to the bag, stirred, and washed by centrifugation. This procedure was repeated four times to obtain a red cell concentrate. The changing ratio measured by the cyanomethemoglobin method was 1.3%, and the hemoglobin yield was 63%.

Next, the red cell concentrate was transferred to a polyethylene container, and an equivalent volume of water for injection was added to hemolyze the red blood cells at a hypotonic condition, and ultrafiltration was performed (ultrafiltering molecular weight: 1000 kDa), thereby removing the stroma component. The resultant solution was degassed and exposed to carbon monoxide gas repeatedly five times to be converted into carbonyl Hb by 98% or more. The resultant carbonyl Hb solution was stirred in a constant temperature bath at 60°C for 12 hours, and subjected to centrifugation (3,000 x g, 30 min.), thereby removing precipitated denatured impure protein. The resultant solution was subjected to ultrafiltration (ultrafiltering molecular weight: 1000 kDa) to obtain a purified hemoglobin solution as a filtered solution. Table 3 shows the properties of the Hb solution obtained by this method.

**Table 3: Properties of Hb solution purified from deep-frozen red cell M.A.P.**

| Item | Measurement |
|---|---|
| Hb concentration (g/dL) | 7 |
| HbCO (%) | 97 |
| MetHb (%) | 2.3 |
| Protein purity in Hb (%) | >99.9 |
| Lipid remaining ratio (%) | <0.01 |
| Hb yield (%) | 55 |

### [Example 5] Method for purifying Hb from deep-frozen and washed red blood cells

A bag (400 ml) of red cell M.A.P. provided by the Japanese Red Cross Blood Center was preserved at 4°C. 30 days after blood extraction, 140 mL of physiological saline was added thereto and subjected to centrifugation (2,000 x g) in the bag at 4°C, thereby removing a supernatant containing buffy coat. In this case, no hemolysis of the red blood cells was recognized. Then, the procedure of adding 140 mL of physiological saline to the bag and performing centrifugation was repeated three times to obtain a red cell concentrate. The red cell concentrate in the bag was immersed in liquid nitrogen to be deep-frozen. After being preserved in a freezer (-80°C) for 60 days, the deep-frozen red cell concentrate was defrosted in a refrigerator (4°C). The changing ratio measured by the cyanomethemoglobin method was 0.9%, and the hemoglobin yield was 90%.

Next, the defrosted red cell concentrate was transferred from the bag to a polyethylene container, and an equivalent volume of water for injection was added to hemolyze the red blood cells at a hypotonic condition, and ultrafiltration was performed (ultrafiltering molecular weight: 1000 kDa), thereby removing the stroma component. The resultant solution was degassed and exposed to carbon monoxide gas repeatedly five times to be converted into carbonyl Hb by 98% or more. The resultant carbonyl Hb solution was stirred in a constant temperature bath at 60°C for 12 hours, and subjected to centrifugation (2,000 x g, 30 min.), thereby removing precipitated denatured impure protein. The resultant solution was subjected to ultrafiltration (ultrafiltering molecular weight: 1000 kDa) to obtain a purified hemoglobin solution as a filtered solution. Table 4 shows the properties of the Hb solution obtained by this method.

**Table 4: Properties of Hb solution purified from deep-frozen and washed red blood cells**

| Item | Measurement |
|---|---|
| Hb concentration (g/dL) | 7 |
| HbCO (%) | 96 |
| MetHb (%) | 1.9 |
| Protein purity in Hb (%) | >99.9 |
| Lipid remaining ratio (%) | <0.01 |
| Hb yield (%) | 80 |

### [Example 6] Method for purifying Hb from deep-frozen and washed red blood cells

Red cell M.A.P. provided by the Japanese Red Cross Blood Center was preserved at 4°C. 36 days after blood extraction, 10 L of the red cell M.A.P. was transferred from the bag to a polyethylene container and subjected to centrifugation (2,000 x g), thereby removing a supernatant containing buffy coat. In this case, much hemolysis of the red blood cells was not recognized. Then, 5 L of physiological saline was added, stirred, and washed by centrifugation. This procedure was repeated four times to obtain a red cell concentrate. Next, the red cell concentrate was deep-frozen in a freezer (-80°C), preserved for 30 days, and defrosted in a refrigerator (4°C). The changing ratio measured by the cyanomethemoglobin method was 0.7%, and the hemoglobin yield was 93%.

Next, an equivalent volume of water for injection was added to the defrosted red cell concentrate to hemolyze the red blood cells at a hypotonic condition, and ultrafiltration was performed (ultrafiltering molecular weight: 1000 kDa), thereby removing the stroma component. The resultant solution was degassed and exposed to carbon monoxide gas repeatedly five times to be converted into carbonyl Hb by 98% or more. The resultant carbonyl Hb solution was stirred in a constant temperature bath at 60°C for 12 hours, and subjected to centrifugation (2,000 x g, 30 min.), thereby removing precipitated denatured impure protein. 0.1 N sodium hydroxide solution was added to the resultant solution, and the mixture solution was adjusted to have a pH of 7.4. The resultant solution was subjected to ultrafiltration (ultrafiltering molecular weight: 1000 kDa) to obtain a purified hemoglobin solution as a filtered solution. Table 5 shows the properties of the Hb solution obtained by this method.

**Table 5: Properties of Hb concentrate solution purified from deep-frozen and washed red blood cells**

| Item | Measurement |
|---|---|
| Hb concentration (g/dL) | 41 |
| HbCO (%) | 98 |
| MetHb (%) | 2.0 |
| Protein purity in Hb (%) | >99.9 |
| Lipid remaining ratio (%) | <0.01 |
| Hb yield (%) | 78 |

### INDUSTRIAL APPLICABILITY

The present invention provides a method for purifying hemoglobin. According to a method of the present invention, by which hemoglobin is deep-frozen and preserved and then purified, Hb in the red blood cells is kept stable. This significantly extends the term in which the red blood cells is usable. Therefore, the present invention is very useful in the field of, for example, emergency medicine.

## Claims

1. A method for purifying hemoglobin from red blood cells, wherein the red blood cells are deep-frozen and preserved, and then purified.

2. A method according to claim 1, wherein the step of deep-freezing and preserving the red blood cells comprises a step of preserving the extracted red blood cells at 4°C through 10°C, a step of washing the red blood cells, a step of deep-freezing the washed red blood cells, and a step of preserving the deep-frozen red blood cells in a frozen state.

3. A method according to claim 2, wherein the term from the red blood cells are extracted until deep-frozen is 1 through 60 days.

4. A method according to claim 2 or 3, wherein the step of deep-freezing the red blood cells and the step of preserving the red blood cells in the frozen state are performed at a temperature of -60°C or lower.
